(19) Europäisches Patentamt
European Patent Office
Office européen des brevets

(11) **EP 0 944 565 B1**

(12) **EUROPÄISCHE PATENTSCHRIFT**

(45) Veröffentlichungstag und Bekanntmachung des Hinweises auf die Patenterteilung:
**31.07.2002 Patentblatt 2002/31**

(21) Anmeldenummer: **97954741.1**

(22) Anmeldetag: **12.12.1997**

(51) Int Cl.7: **C07C 25/22**, C09K 19/32, C09K 19/34, C09K 19/40, C07C 43/225, C07D 213/64, C07D 303/12, C07F 7/08, C07D 213/61, C07C 69/22, C07C 69/75

(86) Internationale Anmeldenummer:
**PCT/EP97/06999**

(87) Internationale Veröffentlichungsnummer:
**WO 98/27038 (25.06.1998 Gazette 1998/25)**

(54) **1,3,5-TRIFLUORNAPHTHALIN-DERIVATE FÜR FLÜSSIGKRISTALLINEN MISCHUNGEN**

1,3,5-TRIFLUORONAPHTHALENE DERIVATIVES FOR LIQUID CRYSTALLINE MIXTURES

DERIVES DE 1,3,5-TRIFLUORONAPHTALENE POUR MELANGES DE CRISTAUX LIQUIDES

(84) Benannte Vertragsstaaten:
**DE**

(30) Priorität: **16.12.1996 DE 19652249**

(43) Veröffentlichungstag der Anmeldung:
**29.09.1999 Patentblatt 1999/39**

(73) Patentinhaber: **Aventis Research & Technologies GmbH & Co. KG**
**65926 Frankfurt am Main (DE)**

(72) Erfinder:
- **MANERO, Javier**
**D-65931 Frankfurt am Main (DE)**
- **SCHMIDT, Wolfgang**
**D-51143 Köln (DE)**

(74) Vertreter: **Isenbruck, Günter, Dr.**
**Patent- und Rechtsanwälte,**
**Bardehle-Pagenberg-Dost-Altenburg-Geissler-Isenbruck**
**Theodor-Heuss-Anlage 12**
**68165 Mannheim (DE)**

(56) Entgegenhaltungen:
**DE-A- 19 517 038** **DE-A- 19 522 195**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 944 565 B1

## Beschreibung

Neben nematischen und cholesterischen Flüssigkristallen werden in jüngerer Zeit auch optisch aktive geneigt smektische (ferroelektrische) Flüssigkristalle in kommerziellen Displayvorrichtungen verwendet.

Clark und Lagerwall konnten zeigen, daß der Einsatz ferroelektrischer Flüssigkristalle (FLC) in sehr dünnen Zellen zu optoelektrischen Schalt-oder Anzeigeelementen führt, die im Vergleich zu den herkömmlichen TN ("twisted nematic")-Zellen um bis zu einem Faktor 1000 schnellere Schaltzeiten haben (siehe z.B. EP-A 0 032 362). Aufgrund dieser und anderer günstiger Eigenschaften, z.B. der bistabilen Schaltmöglichkeit und des nahezu blickwinkelunabhängigen Kontrasts, sind FLCs grundsätzlich für Anwendungsgebiete wie Computerdisplays gut geeignet.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S_A$ und $S^*_C$-Phase läßt sich z.B. erreichen, wenn die Phasenfolge der Flüssigkristallmischung mit abnehmender Temperatur lautet:

$$\text{Isotrop} \rightarrow N^* \rightarrow S_A \rightarrow S^*_C$$

Voraussetzung ist, daß der Pitch (Ganghöhe der Helix) in der N*-Phase sehr groß (größer 10 µm) oder, noch besser, völlig kompensiert ist (siehe z.B. T. Matsumoto et al., Proc. of the 6th Int. Display Research Conf., Japan Display, Sept. 30- Okt. 2, 1986, Tokyo, Japan. S. 468-470; M. Murakami et al., ibid. S. 344 -S. 347). Dies erreicht man z.B., indem man zu der chiralen Flüssigkristallmischung, die in der N*-Phase z.B. eine linksdrehende Helix aufweist, einen oder mehrere optisch aktive Dotierstoffe, die eine rechtsdrehende Helix induzieren, in solchen Mengen hinzugibt, daß die Helix kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Voraussetzung, daß der Pitch in der smektischen C*-Phase wesentlich größer ist als die Dicke des Anzeigeelementes (Mol. Cryst. Liq. Cryst. 1983, 94, 213 und 1984, 114, 151).

Die optische Schaltzeit $\tau$ [µs] ferroelektrischer Flüssigkristallsysteme, die möglichst kurz sein soll, hängt von der Rotationsviskosität des Systems $\gamma$ [mPas], der spontanen Polarisation $P_s$[nC/cm$^2$] und der elektrischen Feldstärke E[V/m] ab nach der Beziehung

$$\tau \sim \frac{\gamma}{P_s \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$ und eine geringe positive oder vorzugsweise negative dielektrische Anisotropie $\Delta\varepsilon$ verlangt (siehe z.B. S.T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktsemiedrigung und zur Verbreiterung der $S_C$-und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Ferroelektrische Flüssigkristallanzeigen lassen sich auch durch Nutzung des DHF (Distorted Helix Formation) -Effektes oder des PSFLCD-Effektes (Pitch Stabilized Ferroelectric Liquid Crystal Display, auch SBF = Short Pitch Bistable Ferroelectric Effect genannt) betreiben. Der DHF-Effekt wurde von B.I. Ostrovski in Advances in Liquid Crystal Research and Applications, Oxford/Budapest 1980, 469ff. beschrieben, der PSFLCD-Effekt ist in DE-A 39 20 625 bzw. EP-A 0 405 346 beschrieben. Zur Nutzung dieser Effekte wird im Gegensatz zum SSFLCD-Effekt ein flüssigkristallines Material mit einem kurzen $S_C$-Pitch benötigt.

Naphthalinderivate zur Verwendung in Flüssigkristallmischungen sind beispielsweise aus der WO-A 92/16

500 bekannt. 1,2,8-Trifluomaphthalinderivate sind aus der DE-A 195 22 195 bekannt.

Da aber die Entwicklung, insbesondere von ferroelektrischen Flüssigkristallmischungen, noch in keiner Weise als abgeschlossen betrachtet werden kann, sind die Hersteller von Displays an den unterschiedlichsten Komponenten für Mischungen interessiert. Dieses u.a. auch deshalb, weil erst das Zusammenwirken der flüssigkristallinen Mischungen mit den einzelnen Bauteilen der Anzeigevorrichtung bzw. der Zellen (z.B. der Orientierungsschicht) Rückschlüsse auf die Qualität auch der flüssigkristallinen Mischungen zuläßt.

Aufgabe der vorliegenden Erfindung war es daher, neue Verbindungen bereitzustellen, die in flüssigkristallinen Mischungen geeignet sind, das Eigenschaftsprofil dieser Mischungen zu verbessern, insbesondere in Mischungen zum Betrieb im Inverse - oder $TV_{(min)}$-Mode.

Es wurde nun gefunden, daß 1,3,5-Trifluornaphthalin-Derivate der Formel (I) in besonderer Weise zum Einsatz in Flüssigkristallmischungen geeignet sind.

Gegenstand der Erfindung sind daher 1,3,5-Trifluornaphthalin-Derivate der Formel (I),

$$R^1(-A^1-M^1)_a(-A^2-M^2)_b-B(-M^3-A^3)_c(-M^4-A^4)_d-R^2 \qquad (I)$$

wobei die Symbole und Indizes folgende Bedeutungen haben:

die Gruppe B ist

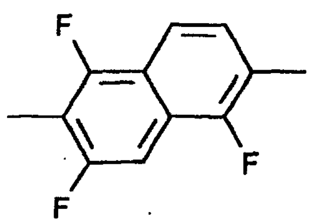

a, b, c, d sind 0 oder 1; mit der Maßgabe, daß die Verbindung der Formel (I) nicht mehr als vier fünf- oder mehrgliedrige Ringsysteme enthält.

$R^1$, $R^2$ sind gleich oder verschieden

a) Wasserstoff,

b) ein geradkettiger oder verzweigter Alkylrest (mit oder ohne asymmetrisches C-Atom) mit 1 bis 16 C-Atomen, wobei

b1) eine oder zwei nicht benachbarte und nicht terminale $CH_2$-Gruppen durch -O-, -CO-O-, -O-CO-, -O-CO-O- oder $-Si(CH_3)_2$ersetzt sein können und/oder

b2) eine $CH_2$-Gruppe duch 1,4-Phenylen oder trans-1,4-Cyclohexylen ersetzt sein kann und/oder

b3) ein oder mehrere H-Atome durch F ersetzt sein können und/oder

b4) die terminale $CH_3$-Gruppe durch eine der folgenden chiralen Gruppen (optisch aktiv oder racemisch) ersetzt sein kann:

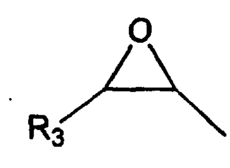

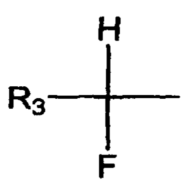

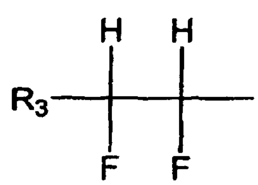

mit der Maßgabe, daß höchstens einer der Reste $R^1$, $R^2$ Wasserstoff ist.

$R^3$ ist ein geradkettiger oder verzweigter Alkylrest (mit oder ohne asymmetrisches Kohlenstoffatom) mit 1 bis 14 C-Atomen, wobei
eine nicht terminale $CH_2$-Gruppe durch -O- ersetzt sein kann

$M^1$, $M^2$, $M^3$, $M^4$ sind gleich oder verschieden -CO-O-, -O-CO-, $-CH_2-O-$, $-O-CH_2-$ oder eine Einfachbindung.

$A^1$, $A^2$, $A^3$, $A^4$ sind gleich oder verschieden 1,4-Phenylen, wobei ein oder zwei H-Atome durch F ersetzt sein können, Pyridin-2,5-diyl, wobei ein H-Atom durch F ersetzt sein kann, Pyrimidin-2,5-diyl, trans-1,4-Cyclohexylen. wobei ein oder zwei H-Atome durch CN und/oder $CH_3$ und/oder F ersetzt sein können.

Ganz besonders bevorzugt sind die folgenden Verbindungen der Formel (Ia) bis (Ii):

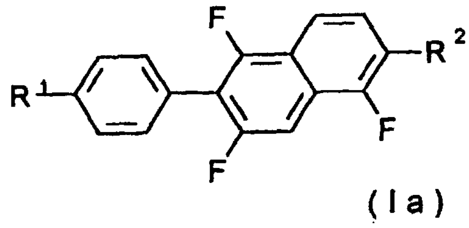
(Ia)

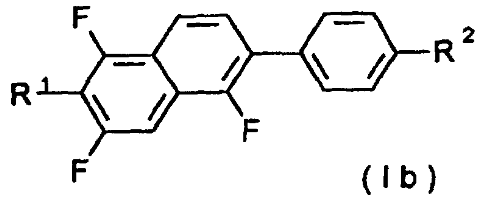
(Ib)

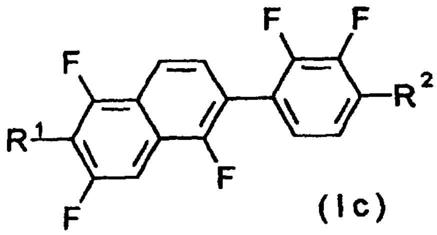
(Ic)

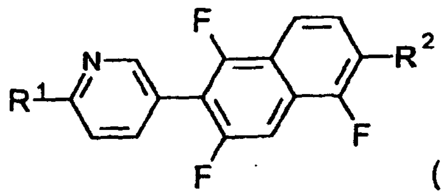
(Id)

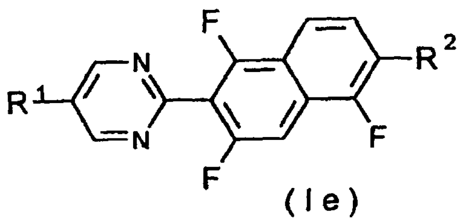
(Ie)

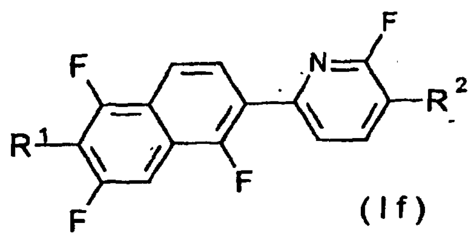
(If)

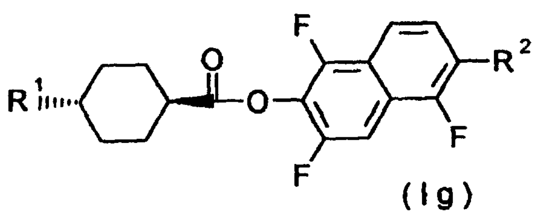
(Ig)

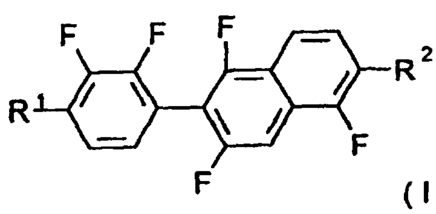
(Ih)

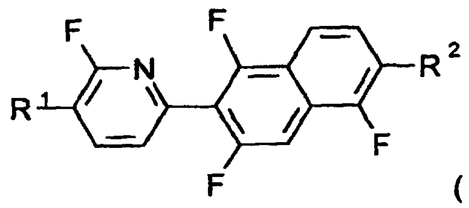

(I i)

wobei $R^1$, $R^2$ die oben angegebenen Bedeutungen haben.

Mit der Bereitstellung von Verbindungen der Formel (I) wird ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

In diesem Zusammenhang besitzen die Verbindungen der Formel (I) einen breiten Anwendungsbereich. In Abhängigkeit von der Auswahl der Substituenten können sie als Basismaterialien dienen, aus denen flüssigkristalline Phasen zum überwiegenden Teil zusammengesetzt sind; es können aber auch Verbindungen der Formel (I) flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Besonders geeignet sind die Verbindungen der Formel (I), um schon in geringen Zumischmengen die dielektrische Anisotropie ($\Delta\varepsilon$) in Richtung auf höhere negative Werte zu beeinflussen.

Die erfindungsgemäßen Verbindungen der Formel (I) eignen sich insbesondere zur Verwendung in FLC-Mischungen für ferroelektrische Schalt- und/oder Anzeigevorrichtungen, die im Inverse-Mode betrieben werden.

Bevorzugt enthalten die Verbindungen der Formel (I) zwei oder mehr fünf- oder mehrgliedrige Ringsysteme.

Die Herstellung der erfindungsgemäßen Verbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch machen.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

Beispielhaft ist in Schema 1 ein Syntheseweg für die Synthese von 1,3,5-Trifluornaphthalin und zu Verbindungen der Formel I angegeben, wobei auch andere Verfahren denkbar und möglich sind.

A)

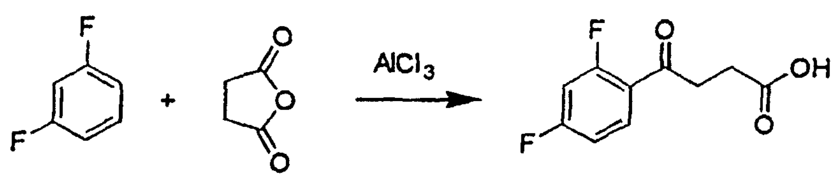


B)

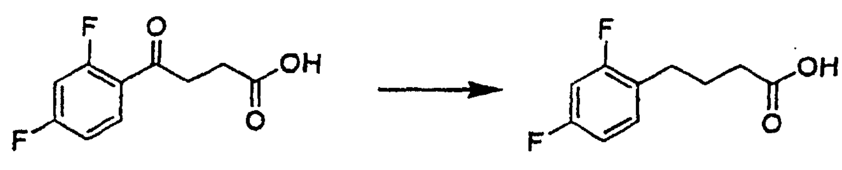

C)

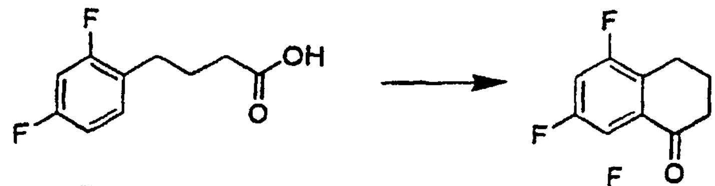

D)

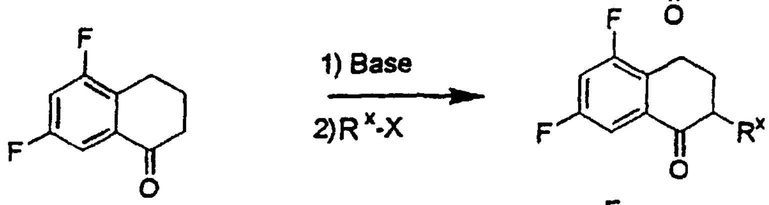


E)

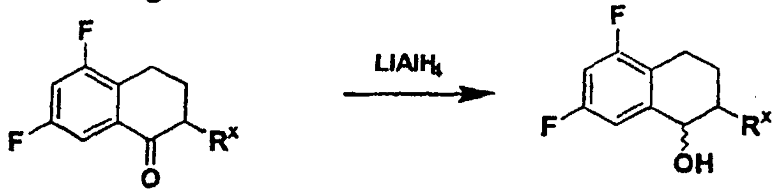


F)

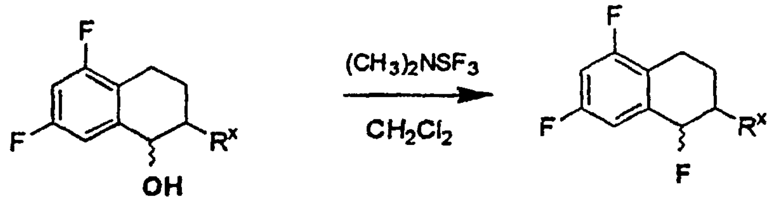


G)

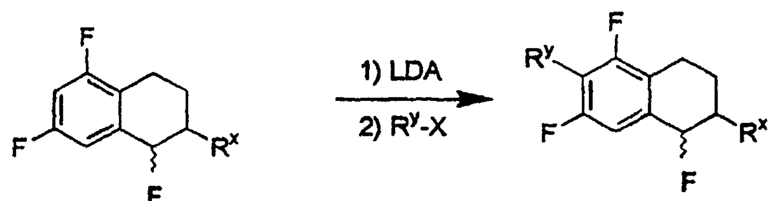


H)

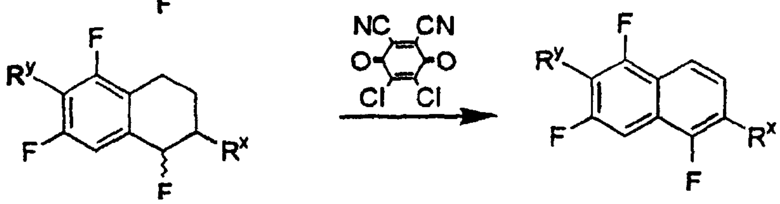

A)-C) J. Med. Chem., 36 (1993) 2810-2816
D) analog Rec. Chem. Prog., 28 (1968) 99
E) analog J. Am. Chem. Soc., 98 (1976) 8114
F) analog J. Org. Chem., 40 (1975) 574
G) analog PCT/GB/01525
H) analog J. Chem. Soc., 1954, 3569

In Schema 2 ist beispielhaft die Synthese von Verbindungen der Formel I mit einer Arylsubstitution in der Position 2 des Naphthalinsystems angegeben.

Schema 2

I)

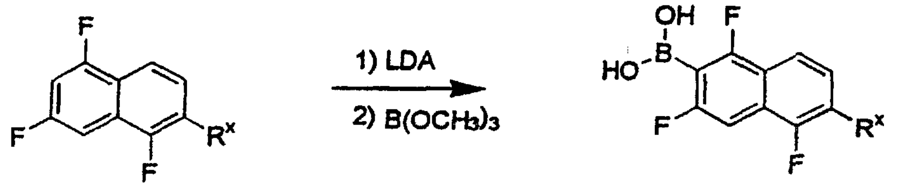


J)

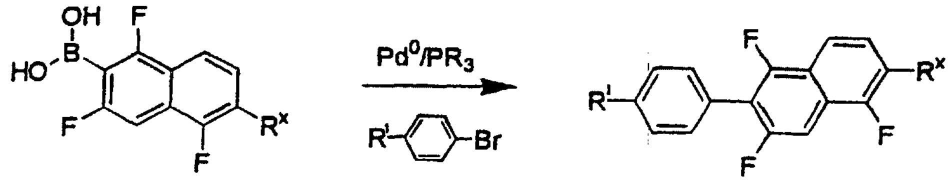


I) analog EP 92901407
J) analog EP 9511158.2

Die Gruppe $R^y$ ist gleich der Gruppierung $R^1(-A^1-M^1)_a(-A^2-M^2)_b$- oder eine geeignete, gegebenenfalls geschützte Vorstufe hiervon, die in späteren Schritten nach an sich bekannten, dem Fachmann geläufigen Methoden in diese Gruppierung überführt werden kann. Die Gruppe $R^X$ ist gleich der Gruppierung $(-M^3-A^3)_c(-M^4-A^4)_d-R^2$ oder eine geeignete, gegebenenfalls geschützte Vorstufe hiervon, die in späteren Schritten nach an sich bekannten, dem Fachmann geläufigen Methoden in diese Gruppierung überführt werden kann. Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch machen.

Beispielsweise sei verwiesen auf DE-A 23 44 732, 24 50 088, 24 29 093, 25 02 94, 26 36 684, 27 01 591 und 27 52 975 für Verbindungen mit 1,4-Cyclohexylen und 1,4-Phenylen-Gruppen; DE-A 26 41 724 für Verbindungen mit Pyrimidin-2,5-diyl-Gruppen; DE-A 40 26 223 und EP-A 03 91 203 für Verbindungen mit Pyridin-2,5-diyl-Gruppen; DE-A 32 31 462 für Verbindungen mit Pyridazin-3,6-diyl-Gruppen; EP-A 309 514 für Verbindungen mit (1,3,4)-Thiadiazol-2-5-diyl-Gruppen; WO-A 92/16500 für Naphthalin-2,6-diyl-Gruppen und EP-A 0 630 903 für Verbindungen mit 1-Sila-1,4-cyclohexylen-Gruppen.

Die Herstellung disubstituierter Pyridine, disubstituierter Pyrazine, disubstituierter Pyrimidine und disubstituierter Pyridazine findet sich beispielsweise auch in den entsprechenden Bänden der Serie "The Chemistry of Heterocyclic Compounds" von A. Weissberger und E.C. Taylor (Herausgeber).

Dioxanderivate werden zweckmäßig durch Reaktion eines entsprechenden Aldehyds (oder eines seiner reaktionsfähigen Derivate) mit einem entsprechenden 1,3-Diol (oder einem seiner reaktionsfähigen Derivate) hergestellt, vorzugsweise in Gegenwart eines inerten Lösungsmittels, wie Benzol oder Toluol, und/oder eines Katalysators, z.B. einer starken Säure, wie Schwefelsäure, Benzol- oder p-Toluolsulfonsäure, bei Temperaturen zwischen etwa 20°C und etwa 150°C, vorzugsweise zwischen 80°C und 120°C. Als reaktionsfähige Derivate der Ausgangsstoffe eignen sich in erster Linie Acetale.

Die genannten Aldehyde und 1,3-Diole sowie ihre reaktionsfähigen Derivate sind zum Teil bekannt, zum Teil können sie ohne Schwierigkeiten nach Standardverfahren der Organischen Chemie aus literaturbekannten Verbindungen hergestellt werden. Beispielsweise sind die Aldehyde durch Oxydation entsprechender Alkohole oder durch Reduktion von Nitrilen oder entsprechenden Carbonsäuren oder ihrer Derivate, die Diole durch Reduktion entsprechender Diester erhältlich.

Verbindungen, worin ein aromatischer Ring durch mindestens ein F-Atom substituiert ist, können auch aus den entsprechenden Diazoniumsalzen durch Austausch der Diazoniumgruppe gegen ein Fluoratom, z.B. nach den Methoden von Balz und Schiemann, erhalten werden.

Was die Verknüpfung der Ringsysteme miteinander angeht, sei beispielsweise verwiesen auf:
N. Miyaura, T. Yanagai und A. Suzuki in Synthetic Communications 11 (1981), 513-519, DE-C 39 30 663, M.J. Sharp, W. Cheng, V. Snieckus in Tetrahedron Letters 28 (1987) 5093; G.W. Gray in J. Chem. Soc. Perkin Trans II 1989, 2041 und Mol. Cryst. Liq. Cryst. 172 (1989) 165, 204 (1991) 43 und 91; EP-A 0 449 015; WO-A 89/12039; WO-A 89/03821; EP-A 0 354 434 und EP-A 0 694 530 für die direkte Verknüpfung von Aromaten und Heteroaromaten; DE-A 32 01 721 für Verbindungen mit $-CH_2CH_2$-Brückengliedern und Koji Seto et al. in Liquid Crystals 8 (1990) 861-870 für Verbindungen mit -C≡C-Brückengliedern.

Ester der Formel (I) können auch durch Veresterung entsprechender Carbonsäuren (oder ihrer reaktionsfähigen Derivate) mit Alkoholen bzw. Phenolen (oder ihren reaktionsfähigen Derivaten), nach der DCC-Methode (DCC = Dicyclohexylcarbodiimid) oder analog DE-A 44 27 198 erhalten werden.

Die entsprechenden Carbonsäuren und Alkohole bzw. Phenole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls, wie Natrium oder Kalium, in Betracht.

Die Veresterung wird vorteilhaft in Gegenwart eines inerten Lösungsmittels durchgeführt. Gut geeignet sind insbesondere Ether, wie Diethylether, Di-nbutylether, THF, Dioxan oder Anisol, Ketone, wie Aceton, Butanon oder Cyclohexanon, Amide, wie DMF oder Phosphorsäurehexamethyltriamid, Kohlenwasserstoffe, wie Benzol, Toluol oder Xylol, Halogenkohlenwasserstoffe, wie Tetrachlorkohlenstoff, Dichlormethan oder Tetrachlorethylen und Sulfoxide, wie Dimethylsulfoxid oder Sulfolan.

Ether der Formel (I) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, Alkylsulfonat oder Dialkylsulfat umgesetzt werden, zweckmäßig in einem inerten Lösungsmittel, wie Aceton, 1,2-Dimethoxyethan, DMF oder Dimethylsulfoxid, oder auch mit einem Überschuß an wäßriger oder wäßrig-alkoholischer NaOH oder KOH bei Temperaturen zwischen etwa 20° und 100°C.

Was die Synthese spezieller Reste $R^1$ und $R^2$ angeht, sei zusätzlich beispielsweise verwiesen auf
EP-B 0 355 008 für Verbindungen mit siliziumhaltigen Seitenketten,
EP-B 0 292 954 für optisch aktive Verbindungen mit Oxiranestereinheit,
EP-B 0 263 437 für optisch aktive Verbindungen mit Oxiranethereinheit,
EP-B 0 361 272 für optisch aktive Verbindungen mit Dioxolanestereinheit,
EP-B 0 351 746 für optisch aktive Verbindungen mit Dioxolanethereinheit,
US 5,051,506 für optisch aktive Verbindungen mit 2,3-Difluoralkyloxy-Einheit,
US 4,798,680 für optisch aktive Verbindungen mit 2-Fluoralkyloxy-Einheit,
US 4,855,429 für optisch aktive Verbindungen mit $\alpha$-Chlorcarbonsäure-Einheit,
EP-A 0 552 658 für Verbindungen mit Cyclohexylpropionsäureresten,
EP-A 0 318 423 für Verbindungen mit Cyclopropylgruppen in der Seitenkette.

Die erfindungsgemäßen Verbindungen der Formel (I) eignen sich vorzugsweise für den Einsatz in smektischen und nematischen Flüssigkristallmischungen, im Falle von nematischen Mischungen vorzugsweise für "Active Matrix Displays" (AM-LCD) (siehe z.B. C. Prince, Seminar Lecture Notes, Volume I, p. M-3/3-M-22, SID International Symposium 1997, B.B. Bahadur, Liquid Crystal Applications and Uses, Vol 1, p. 410, World Scientific Publishing, 1990, E. Lüder, Recent Progress of AM LCD's, Proceedings of the 15th International Displays Research Conference, 1995, p. 9-12) und "in-plane-switching Displays" (IPS-LCD), im Falle von smektischen Flüssigkristallmischungen vorzugsweise für chirale geneigt smektische (ferroelektrische oder antiferroelektrische) Displays, für ECB-Displays (Electrically Controlled Birefringence) und für elektrokline Displays.

Gegenstand der Erfindung ist auch die Verwendung von Verbindungen der Formel (I) in Flüssigkristallmischungen, vorzugsweise smektischen und nematischen, besonders bevorzugt ferroelektrischen.
Insbesondere bevorzugt ist die Verwendung in ferroelektrischen Flüssigkristallmischungen, die im Inverse-Mode betrieben werden.

Weiterhin Gegenstand der Erfindung sind Flüssigkristallmischungen, vorzugsweise smektische und nematische, besonders bevorzugt ferroelektrische und antiferroelektrische, insbesondere ferroelektrische, enthaltend eine oder mehrere Verbindungen der Formel (I).

Die erfindungsgemäßen smektischen oder nematischen Flüssigkristallmischungen eignen sich vorzugsweise für den Einsatz in elektrooptischen Displays, im Falle von nematischen Mischungen besonders für "Active Matrix Displays" und "In-plane-switching Displays" (IPS-LCD), im Falle von smektischen Flüssigkristallmischungen für ECB-Displays (Electrically Controlled Birefringence), für elektrokline Displays und chirale geneigt smektische (ferroelektrische oder antiferroelektrische) Displays.

Die erfindungsgemäßen Flüssigkristallmischungen enthalten im allgemeinen 2 bis 35, vorzugsweise 2 bis 25, besonders bevorzugt 2 bis 20 Komponenten.

Sie enthalten im allgemeinen 0,01 bis 80 Gew.-%, vorzugsweise 0,1 bis 60 Gew.-%, besonders bevorzugt 0,1 bis 30 Gew.-%, an einer oder mehreren, vorzugsweise 1 bis 10, besonders bevorzugt 1 bis 5, ganz besonders

bevorzugt 1 bis 3, der erfindungsgemäßen Verbindungen der Formel (I).

Weitere Komponenten von Flüssigkristallmischungen, die erfindungsgemäße Verbindungen der Formel (I) enthalten, werden vorzugsweise ausgewählt aus den bekannten Verbindungen mit smektischen und/oder nematischen und/oder cholesterischen Phasen. Dazu gehören z. B.:

- Derivate des Phenylpyrimidins, wie beispielsweise in WO-A 86/06401 und US-4 874 542 beschrieben,
- metasubstituierte Sechsringaromaten, wie beispielsweise in EP-A 0 578 054 beschrieben,
- Siliziumverbindungen, wie beispielsweise in EP-A 0 355 008 beschrieben,
- mesogene Verbindungen mit nur einer Seitenkette, wie beispielsweise in EP-A 0 541 081 beschrieben,
- Hydrochinonderivate, wie beispielsweise in EP-A 0 603 786 beschrieben,
- Phenylbenzoate, wie beispielsweise bei P. Keller, Ferroelectrics 1984, 58, 3 und J. W. Goodby et al., Liquid Crystals and Ordered Fluids, Bd. 4, New York 1984 beschrieben, und
- Thiadiazole, wie beispielsweise in EP-A 0 309 514 beschrieben.

Als chirale, nicht racemische Dotierstoffe kommen beispielsweise in Frage:

- optisch aktive Phenylbenzoate, wie beispielsweise bei P. Keller, Ferroelectrics 1984, 58, 3 und J. W. Goodby et al., Liquid Crystals and Ordered Fluids, Bd. 4, New York 1984 beschrieben,
- optisch aktive Oxiranether, wie beispielsweise in EP-A 0 263 437 und WO-A 93/13093 beschrieben,
- optisch aktive Oxiranester, wie beispielsweise in EP-A 0 292 954 beschrieben,
- optisch aktive Dioxolanether, wie beispielsweise in EP-A 0 351 746 beschrieben,
- optisch aktive Dioxolanester, wie beispielsweise in EP-A 0 361 272 beschrieben,
- optisch aktive Tetrahydrofuran-2-carbonsäureester, wie beispielsweise in EP-A 0 355 561 beschrieben, und
- optisch aktive 2-Fluoralkylether, wie beispielsweise in EP-A 0 237 007, EP-A 0 428 720 und US-5,051,506 beschrieben.

Geeignete weitere Mischungskomponenten sind insbesondere in der internationalen

Patentanmeldung WO-A 97/04039 aufgeführt, auf die hiermit ausdrücklich Bezug genommen wird.

Bevorzugte weitere Komponenten von FLC-Mischungen, die im Inverse-Mode eingesetzt werden, sind:

Phenanthrenderivate der Formel (II),

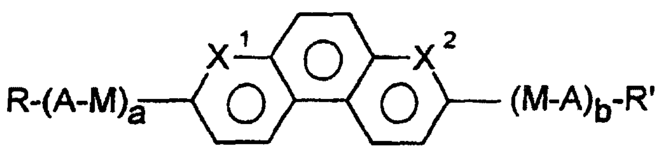

(II)

Fluorpyridine der Formel (III),

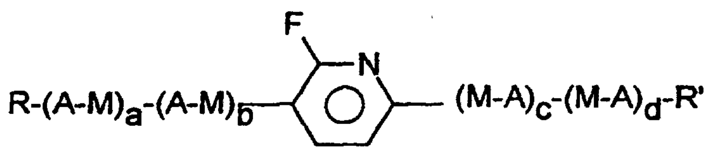

(III)

Difluorphenylenderivate der Formel (IV)

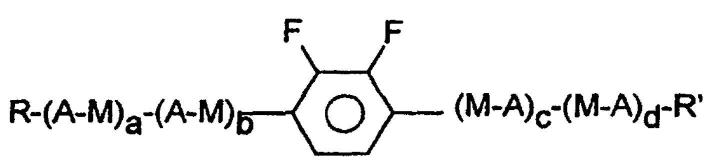

(IV)

Metasubstituierte aromatische Verbindungen der Formel (V)

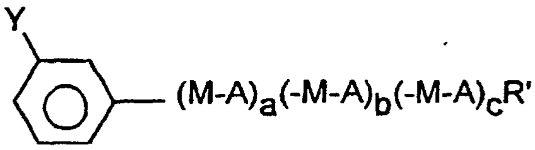

( V )

4-Cyanocyclohexyle der Formel (VI)

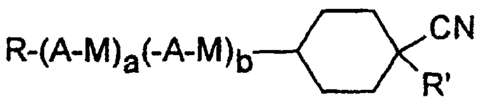

( VI )

1,3,4-Thiadiazole der Formel (VII),

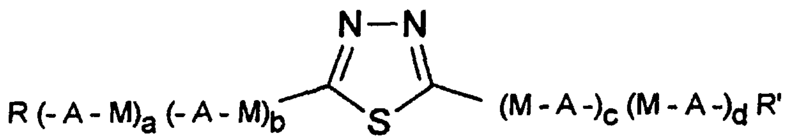

( VII )

wobei die Symbole und Indizes die folgenden Bedeutungen haben:
$X^1$, $X^2$ sind gleich oder verschieden unabhängig voneinander CH, CF oder N;
Y ist F, $CF_3$ oder R;
R, R' haben, gleich oder verschieden, unabhängig voneinander die gleichen Bedeutungen wie $R^1$, $R^2$ in Formel (I);
A, M haben, gleich oder verschieden, unabhängig voneinander die gleichen Bedeutungen wie in Formel (I) und
a,b,c,d sind gleich, oder verschieden, unabhängig voneinander 0 oder 1, mit der Maßgabe, daß die Verbindungen nicht mehr als vier Ringsysteme enthalten dürfen und, mit Ausnahme der Formel (II), mindestens zwei Ringsysteme enthalten müssen.

Die Mischungen wiederum können Anwendung finden in elektrooptischen oder vollständig optischen Elementen, z. B. Anzeigeelementen, Schaltelementen, Lichtmodulatoren, Elementen zur Bildbearbeitung und/oder Signalverarbeitung oder allgemein im Bereich der nichtlinearen Optik.

Femer sind die Mischungen für Feldbehandlung, d. h. zum Betrieb in der Quasi-Bookshelf-Geometrie (QBG) (siehe z. B. H. Rieger et al., SID 91 Digest (Anaheim) 1991, 396) geeignet.

Die erfindungsgemäßen ferroelektrischen Flüssigkristallmischungen eignen sich insbesondere zum Betrieb im sogenannten Inverse- oder $\tau V_{(min)}$-Mode (siehe z. B.: J. C. Jones, M. J. Towler, J. R. Hughes, Displays 1993, 14, Nr. 2, 86-93; M. Koden, Ferroelectrics 1996, 179, 121-129).

Flüssigkristalline Mischungen, die Verbindungen der allgemeinen Formel (I) enthalten, sind besonders für die Verwendung in elektrooptischen Schalt- und Anzeigevorrichtungen (Displays) geeignet. Diese Displays sind üblicherweise so aufgebaut, daß eine Flüssigkristallschicht beiderseitig von Schichten eingeschlossen ist, die üblicherweise, in dieser Reihenfolge ausgehend von der LC-Schicht, mindestens eine Orientierungsschicht, Elektroden und eine Begrenzungsscheibe (z. B. aus Glas) sind. Darüberhinaus können sie Abstandshalter, Kleberahmen, Polarisatoren sowie für Farbdisplays dünne Farbfilterschichten enthalten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (siehe z. B. E. Kaneko, "Liquid Crystal TV Displays: Principles and Applications of Liquid Crystal Displays", KTK Scientific Publishers, 1987).

Ein weiterer Gegenstand der Erfindung ist daher eine Schalt- und/oder Anzeigevorrichtung, vorzugsweise eine smektische oder nematische, insbesondere eine ferroelektrische, enthaltend eine Flüssigkristallmischung, die eine oder mehrere Verbindungen der Formel (I) enthält.
Bei Vorrichtungen, die eine nematische Flüssigkristallmischung enthalten sind "Active Matrix Displays" und "In-plane-switching Displays" (IPS-LCD) bevorzugt.

Bei Vorrichtungen, die eine smektische Flüssigkristallmischung enthalten, sind ECB-Displays (Electrically

Controlled Birefringence), elektrokline Displays und chirale geneigt smektische (ferroelektrische oder antiferroelektrische) Displays bevorzugt.

Solche Vorrichtungen können beispielsweise als Computerdisplays oder in Chipkarten Anwendung finden.

Vorzugsweise wird eine erfindungsgemäße ferroelektrische Schalt- und/oder Anzeigevorrichtung im Normal- oder Inverse-Mode betrieben.

Durch Multiplexansteuerung betriebene ferroelektrische Schalt- und/oder Anzeigevorrichtungen können unter anderem auf zwei unterschiedliche Weisen betrieben werden, die sogenannte normale (Normal-Mode) oder die sogenannte inverse (Inverse Mode auch $\tau V_{(min)}$-Mode). Der Unterschied zwischen beiden liegt im Ansteuerschema und in den verschiedenen Anforderungen an den dielektrischen Tensor des FLC-Materials, d. h. der FLC-Mischung. Einen Überblick geben z. B. J. C. Jones et al. in Displays 1993, 14, Nr. 2, 86-93 im folgenden als "Jones" bezeichnet, und M. Koden in Ferroelectrics 1996, 179, 121-129, sowie die dort aufgeführte Literatur.

Die Schaltcharakteristika einer FLC-Vorrichtung können im allgemeinen durch ein Diagramm dargestellt werden, bei dem die Treiberspannung (V) horizontal und die Breite der Ansteuerpulse ($\tau$, Zeit) vertikal aufgetragen sind (siehe z. B. Jones, Abb. 4.8, 10 und 11).

Eine Schaltkurve wird experimentell bestimmt und teilt die V, $\tau$-Fläche in einen schaltenden- und einen nicht schaltenden Bereich. Üblicherweise verkürzt sich bei Erhöhung der Spannung die Pulsbreite. Dieses Verhalten kennzeichnet den sogenannten Normal-Mode (siehe z. B. Jones, Abb. 4).

Bei geeigneten Materialien weist die $V\tau$-Kurve jedoch ein Minimum auf (bei der Spannung $V_{(min)}$), wie z. B. bei Jones in den Abbildungen 8, 10 und 11 zu sehen. Dieses Minimum kommt durch Überlagerung von dielektrischer und ferroelektrischer Verdrillung zustande. FLC-Vorrichtungen werden im Inverse-Mode betrieben, wenn die Summe der Zeilen- und Spalten-Treiberspannung im Arbeitstemperaturbereich höher als das Minimum auf der $V\tau$-Kurve sind, d. h. $V_{(Zeile)} + V_{(Spalte)} > V_{(min)}$.

In der vorliegenden Anmeldung sind verschiedene Dokumente zitiert, beispielsweise um das technische Umfeld der Erfindung zu illustrieren. Auf alle diese Dokumente wird hiermit ausdrücklich Bezug genommen; sie gelten durch Zitat als Bestandteil der vorliegenden Anmeldung.
Auf den Inhalt der deutschen Patentanmeldung 196 52 249.8, deren Priorität die vorliegende Anmeldung beansprucht, sowie auf die Zusammenfassung der vorliegenden Anmeldung wird hiermit ausdrücklich Bezug genommen; sie gelten durch Zitat als Bestandteil der vorliegenden Anmeldung.

Die Erfindung wird durch die nachfolgenden Beispiele weiter erläutert, ohne sie dadurch beschränken zu wollen.

Beispiel 1:

1,3,5-Trifluor-2-(4-hexyloxyphenyl)-6-octylnaphthalin

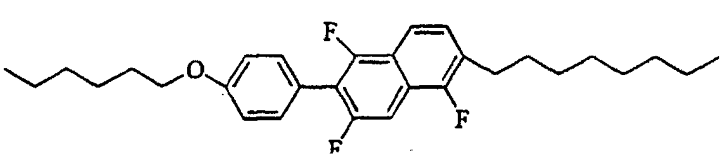

Aus 1,3,5-Trifluor-6-octyl-naphthalin-2-boronsäure und 4-Hexyloxybrombenzol mittels Suzuki-Kopplung.

Beispiel 2:

1,3,5-Trifluor-6-hexyl-2-(4-octylphenyl)naphthalin

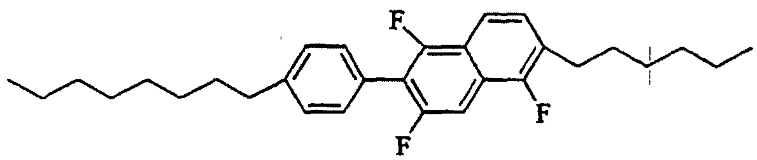

Aus 1,3,5-Trifluor-6-hexylnaphthalin-2-boronsäure und 4-Octylbrombenzol mittels Suzuki-Kopplung.

Beispiel 3:

2-Hexyloxy-5-(1,3,5-trifluor-6-octylnaphthalin-2-yl)pyridin

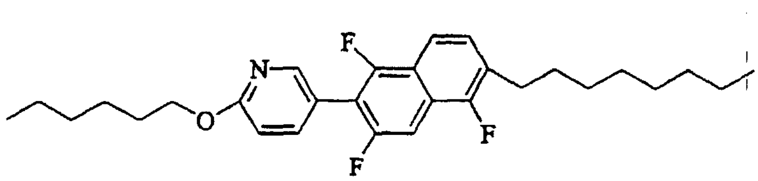

Aus 1,3,5-Trifluor-6-octylnaphthalin-2-boronsäure und 5-Brom-2-hexyloxy-pyridin mittels Suzuki-Kopplung.

Beispiel 4:

1,3,5-Trifluor-2-(2-fluoroctyloxy)-6-(4-hexylphenyl)naphthalin

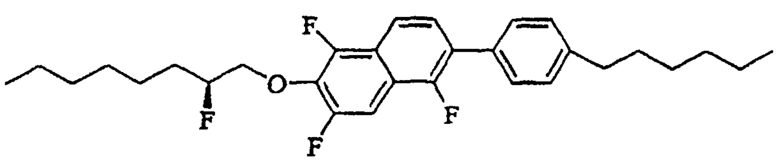

Aus 1,3,5-Trifluor-6-(4-hexyl-phenyl)-naphthalin-2-ol und 2-(S)-Fluor-octan-1-ol mittels Mitsunobu-Reaktion.

Beispiel 5:

2-Butyl-3-[6-(2,3-difluor-4-hexylphenyl)-1,3,5-trifluomaphthalin-2-yloxymethyl]oxiran

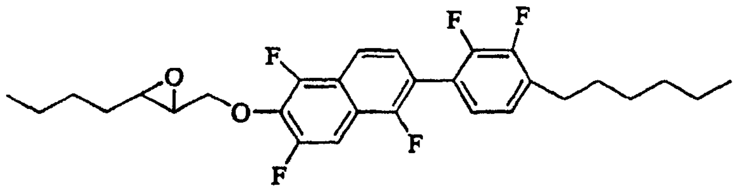

Aus 6-(2,3-Difluor-4-hexylphenyl)-1,3,5-trifluornaphthalin-2-ol und (3-Butyloxiranyl)methanol mittels Mitsunobu-Reaktion.

Beispiel 6:

2-{6-[4-(Butyldimethylsilanyl)butyl]-1,3,5-trifluomaphthalin-2-yl}-5-octyloxy-pyrimidin

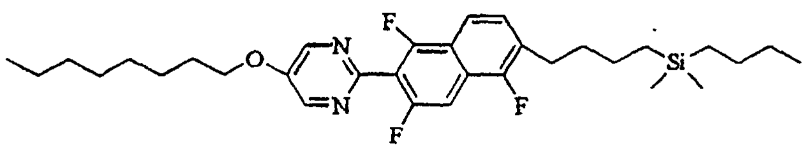

Aus 2-{6-[4-(Butyldimethylsilanyl)-butyl]-1,3,5-trifluomaphthalin-2-yl}-pyrimidin-5-ol und Octylbromid mittels Ethersynthese nach Williamson.

Beispiel 7:

Octansäure 1,3,5-trifluor-6-(4-hexyloxyphenyl)naphthalin-2-yl ester

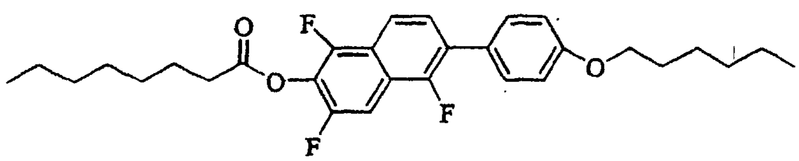

Aus 1,3,5-Trifluor-6-(4-hexyloxyphenyl)naphthalin-2-ol und Octansäure mittels Veresterung mit Dicyclohexylcarbodiimid.

Beispiel 8:

2-Fluoro-3-hexyl-6-[1,5,7-trifluor-6-(8-methyldecyloxy)naphthalin-2-yl]pyridin

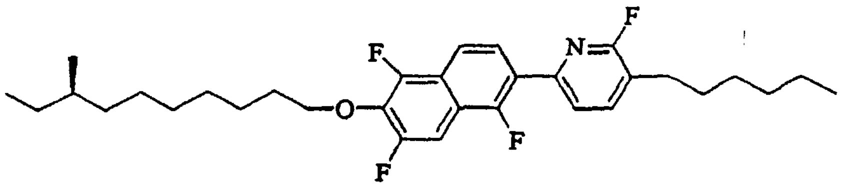

Aus 1,3,5-Trifluor-6-(6-fluor-5-hexylpyridin-2-yl)naphthalin-2-ol und 8-Methyldecan-1-ol mittels Mitsunobu-Reaktion.

Beispiel 9:

4-Pentylcyclohexancarbonsäure 1,3,5-trifluor-6-hexylnaphthalin-2-yl ester

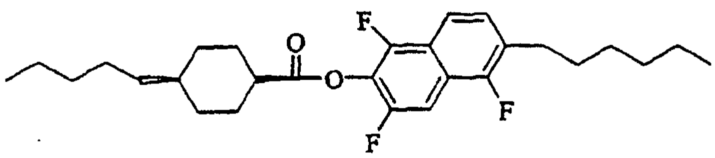

Aus 4-trans-Pentylcyclohexancarbonsäure und 1,3,5-Trifluor-6-hexylnaphthalin-2-ol.

## Patentansprüche

1. 1,3,5-Trifluornaphthalin-Derivat der Formel (I),

$$R^1(-A^1-M^1)_a(-A^2-M^2)_b-B(-M^3-A^3)_c(-M^4-A^4)_d-R^2 \qquad (I)$$

wobei die Symbole und Indizes folgende Bedeutungen haben:

die Gruppe B ist

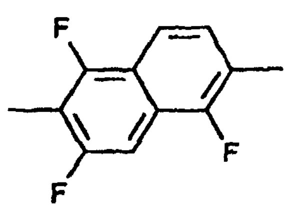

$R^1$, $R^2$ sind gleich oder verschieden

a) Wasserstoff,
b) ein geradkettiger oder verzweigter Alkylrest (mit oder ohne asymmetrisches C-Atom) mit 1 bis 16 C-Atomen, wobei

b1) eine oder zwei nicht benachbarte und nicht terminale $CH_2$-Gruppen durch -O-, -CO-O-, -O-CO-, -O-CO-O- oder - $Si(CH_3)_2$- ersetzt sein können und/oder
b2) eine $CH_2$-Gruppe durch 1,4-Phenylen oder trans-1,4-Cyclohexylen ersetzt sein kann und/oder
b3) ein oder mehrere H-Atome durch F ersetzt sein können und/oder
b4) die terminale $CH_3$-Gruppe durch eine der folgenden chiralen Gruppen (optisch aktiv oder racemisch) ersetzt sein kann:

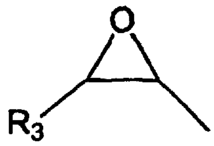
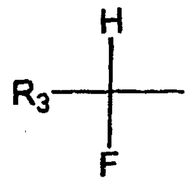
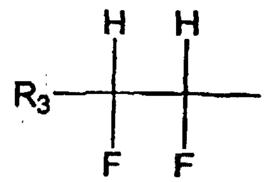

mit der Maßgabe, daß nur einer der Reste $R^1$, $R^2$ Wasserstoff sein kann;
$R^3$, ist
ein geradkettiger oder verzweigter Alkylrest (mit oder ohne asymmetrisches Kohlenstoffatom) mit 1 bis 14 C-Atomen, wobei eine nicht terminale $CH_2$-Gruppe durch -O- ersetzt sein kann
$M^1$, $M^2$, $M^3$, $M^4$ sind gleich oder verschieden -CO-O-, -O-CO-, $-CH_2-O-$, $-O-CH_2-$ oder eine Einfachbindung;
$A^1$, $A^2$, $A^3$, $A^4$ sind gleich oder verschieden 1,4-Phenylen, wobei ein oder zwei H-Atome durch F ersetzt sein können, Pyridin-2,5-diyl, wobei ein H-Atom durch F ersetzt sein kann, Pyrimidin-2,5-diyl, trans-1,4-Cyclohexylen, wobei ein oder zwei H-Atome durch CN und/oder $CH_3$ und/oder F ersetzt sein können;
a, b, c, d sind 0 oder 1; mit der Maßgabe, daß die Verbindung der Formel (I) nicht mehr als vier fünf- oder mehrgliedrige Ringsysteme enthält.

**2.** 1,3,5-Trifluornaphthalin-Derivat gemäß Anspruch 1, ausgewählt aus der Gruppe (Ia) - (Ii):

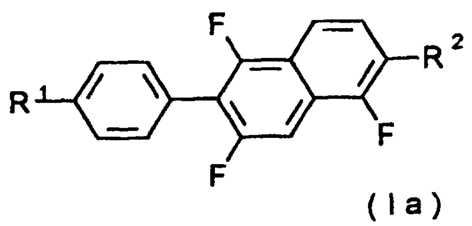
(Ia)

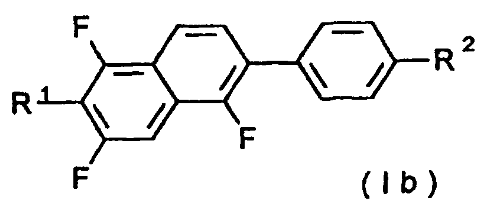
(Ib)

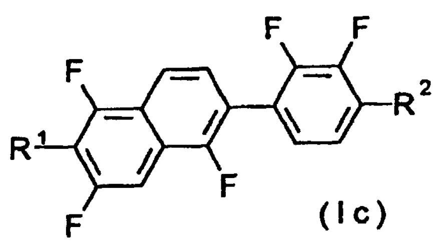
(Ic)

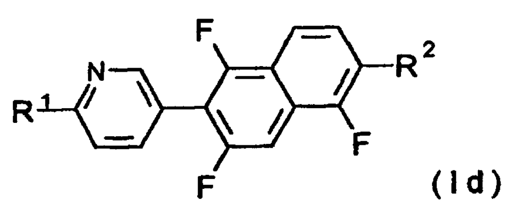
(Id)

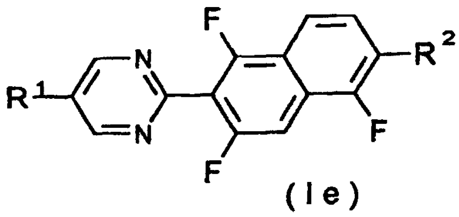
(Ie)

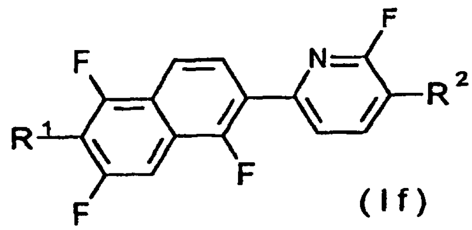
(If)

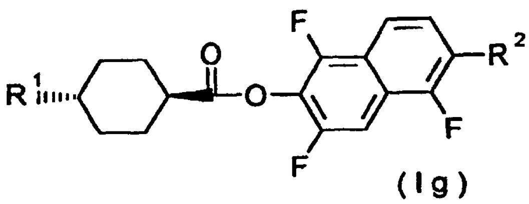
(Ig)

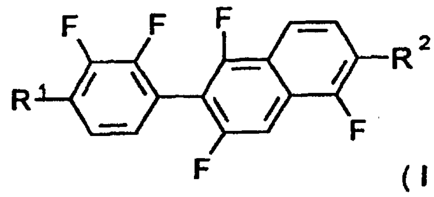
(Ih)

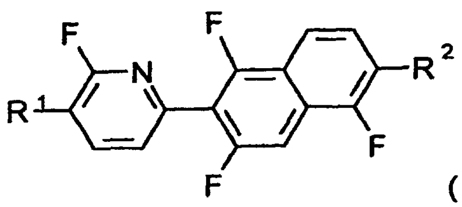
(Ii)

wobei $R^1$, $R^2$ die in der Formel (I) in Anspruch 1 angegebenen Bedeutungen haben, mit der Maßgabe, daß höchstens einer der Reste $R^1$, $R^2$ Wasserstoff sein kann.

3. Verwendung von 1,3,5-Trifluornaphthalin-Derivaten gemäß einem der Ansprüche 1 oder 2 als Komponenten flüssigkristalliner Mischungen.

4. Flüssigkristallmischung, enthaltend ein oder mehrere 1,3,5-Trifluornaphthalin-Derivate gemäß einem der Ansprüche 1 bis 3.

5. Flüssigkristallmischung gemäß Anspruch 4, **dadurch gekennzeichnet, daß** sie ferroelektrisch ist.

6. Flüssigkristallmischung gemäß Anspruch 4 oder 5, **dadurch gekennzeichnet, daß** sie 0,01 bis 80 Gew.-% an einem oder mehreren 1,3,5-Trifluornaphthalin-Derivaten der Formel (I) enthält.

7. Ferroelektrische Schalt- und/oder Anzeigevorrichtung, enthaltend eine ferroelektrische Flüssigkristallmischung gemäß Anspruch 5 oder 6.

8. Ferroelektrische Schalt- undloder Anzeigevorrichtung gemäß Anspruch 7, **dadurch gekennzeichnet, daß** sie im $\tau V_{min}$-Mode betrieben wird.

## Claims

1. A 1,3,5-trifluoronaphthalene derivative of the formula (I)

$$R^1(-A^1-M^1)_a(-A^2-M^2)_b-B(-M^3-A^3)_c(-M^4-A^4)_d-R^2 \quad (I)$$

in which the symbols and indices have the following meanings:

group B is

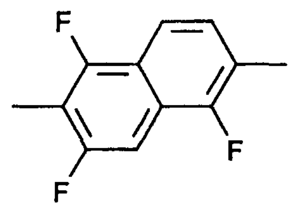

$R^1$ and $R^2$ are identical or different and are

a) hydrogen,
b) a straight-chain or branched alkyl radical (with or without an asymmetric carbon atom) having 1 to 16 carbon atoms, where

b1) one or two nonadjacent and nonterminal $CH_2$ groups can be replaced by -O-, -CO-O-, -O-CO-, -O-CO-O- or $-Si(CH_3)_2-$, and/or
b2) one $CH_2$ group can be replaced by 1,4-phenylene or trans-1,4-cyclohexylene, and/or
b3) one or more H atoms can be replaced by F, and/or
b4) the terminal $CH_3$ group can be replaced by one of the following chiral groups (optically active or racemic):

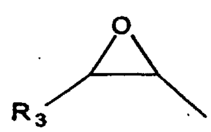

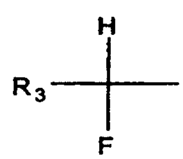

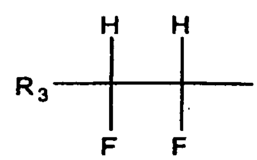

with the proviso that only one of the radicals $R^1$ and $R^2$ can be hydrogen;

$R^3$ is a straight-chain or branched alkyl radical (with or without an asymmetric carbon atom) having 1 to 14 carbon atoms, where one nonterminal $CH_2$ group can be replaced by -O-,

$M^1$, $M^2$, $M^3$ and $M^4$ are identical or different and are -CO-O-, -O-CO-, $-CH_2-O-$, $-O-CH_2-$ or a single bond;

$A^1$, $A^2$, $A^3$, $A^4$ are identical or different and are 1,4-phenylene in which one or two H atoms can be replaced by F, pyridine-2,5-diyl in which one H atom can be replaced by F, pyrimidine-2,5-diyl, trans-1,4-cyclohexylene in which one or two H atoms can be replaced by CN and/or $CH_3$ and/or F;

a, b, c and d are 0 or 1; with the proviso that the compound of the formula (I) contains no more than four ring systems having five or more members.

**2.** A 1,3,5-trifluoronaphthalene derivative as claimed in claim 1, which is selected from the group consisting of (Ia) - (Ii):

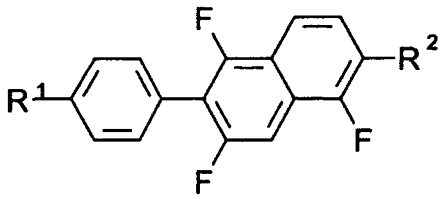

(Ia)

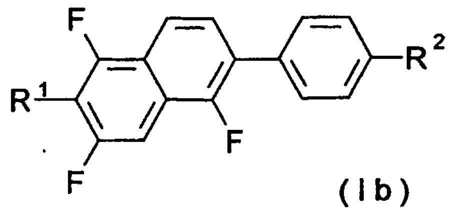

(Ib)

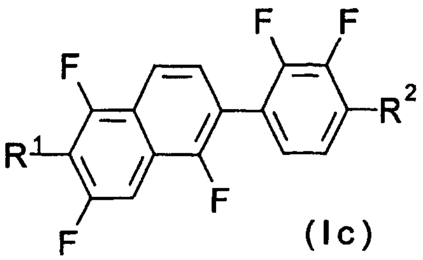

(Ic)

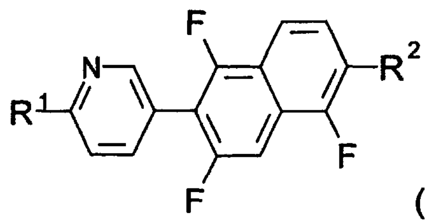

(Id)

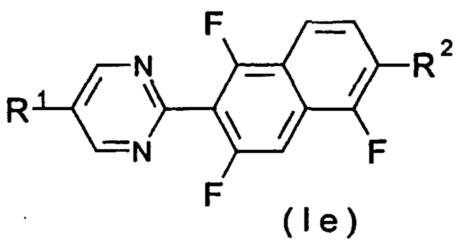

(Ie)

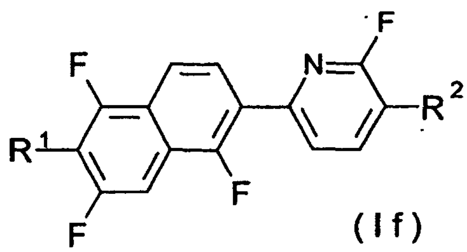

(If)

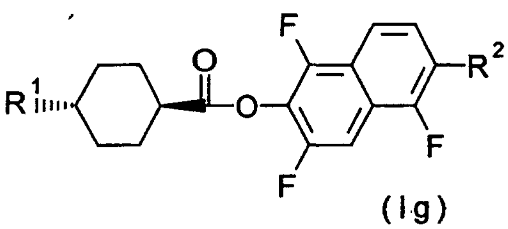

(Ig)

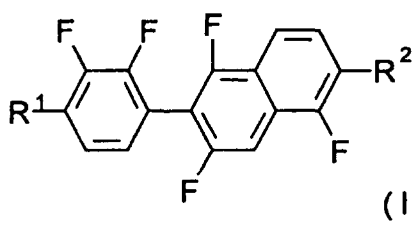

(Ih)

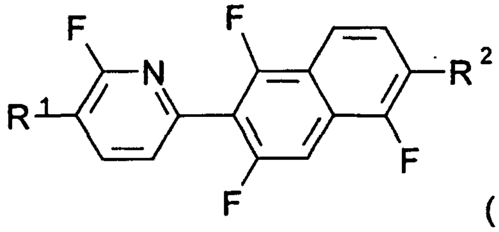

(I i)

in which $R^1$ and $R^2$ have the meanings stated in formula (I) in claim 1, with the proviso that at most one of the radicals $R^1$ and $R^2$ can be hydrogen.

**3.** The use of a 1,3,5-trifluoronaphthalene derivative as claimed in one of claims 1 or 2 as a component of liquid-crystalline mixtures.

**4.** A liquid-crystal mixture comprising one 1,3,5-trifluoronaphthalene derivatives as claimed in one of claims 1 to 3.

**5.** A liquid-crystal mixture as claimed in claim 4, which is ferroelectric.

**6.** A liquid-crystal mixture as claimed in claim 4 or 5, which contains from 0.01 to 80% by weight of one or more 1,3,5-trifluoronaphthalene derivatives of the formula (I).

**7.** A ferroelectric switching and/or display device comprising a ferroelectric liquid-crystal mixture as claimed in claim 5 or 6.

**8.** A ferroelectric switching and/or display device as claimed in claim 7, which is operated in the $\tau V_{min}$ mode.

**Revendications**

**1.** Dérivé 1,3,5-trifluoronaphtalène de formule (I),

$$R^1(-A^1-M^1)_a(-A^2-M^2)_b-B(-M^3-A^3)_c(-M^4-A^4)_d-R^2 \qquad (I)$$

dans laquelle les symboles et indices ont les significations suivantes :

le groupe B est

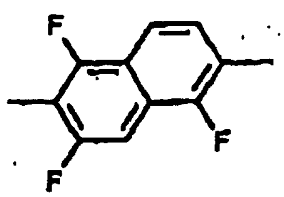

$R^1$, $R^2$ sont identiques ou différents

a) un atome d'hydrogène,
b) un groupe alkyle à chaîne linéaire ou ramifiée (avec ou sans atome de carbone asymétrique) ayant de 1 à 16 atomes de carbone, dans lequel

b1) un ou deux groupes $CH_2$ non voisins et non terminaux peuvent être remplacés par -O-, -CO-O-, -O-CO-, -O-CO-O- ou $-Si(CH_3)_2-$ et/ou
b2) un groupe $CH_2$ peut être remplacé par un 1,4-phénylène ou trans-1,4-cyclohexylène et/ou
b3) un ou plusieurs atomes H peuvent être remplacés par F et/ou
b4) le groupe $CH_3$ terminal peut être remplacé par un des groupes chiraux suivants (optiquement actifs ou racémiques) :

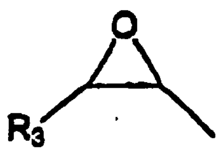

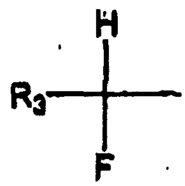

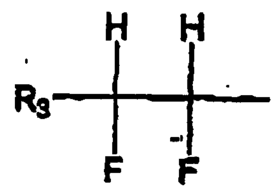

avec la condition, que seulement un des groupes $R^1$, $R^2$ puisse être un atome d'hydrogène ;

$R^3$ est un groupe alkyle à chaîne linéaire ou ramifiée (avec ou sans atome de carbone asymétrique) ayant de 1 à 14 atomes de carbone, dans lequel un groupe $CH_2$ non terminal peut être remplacé par -O-.
$M^1$, $M^2$, $M^3$, $M^4$ sont identiques ou différents -CO-O-, -O-CO-, $-CH_2-O-$, $-O-CH_2-$ ou une liaison simple ;
$A^1$, $A^2$, $A^3$, $A^4$ sont identiques ou différents un groupe 1,4-phénylène, dans lequel un . ou deux atomes H peuvent être remplacés par F, pyridine-2,5-diyle, dans lequel un atome H peut être remplacé par F, pyrimidine-2,5-diyle, trans-1,4-cyclohexylène, dans lequel un ou deux atomes H peuvent être remplacés par CN et/ou $CH_3$ et/ou F ;
a, b, c, d sont 0 ou 1 ; avec la condition que le composé de formule (I) ne contienne pas plus de quatre systèmes cycliques à cinq chaînons ou plus.

**2.** Dérivé 1,3,5-trifluoronaphtalène selon la revendication 1 choisi parmi le groupe (Ia)-(Ii) :

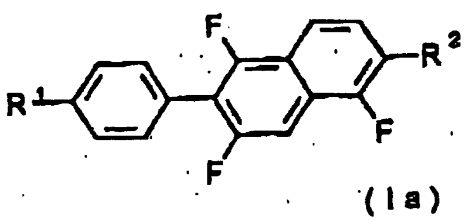
(Ia)

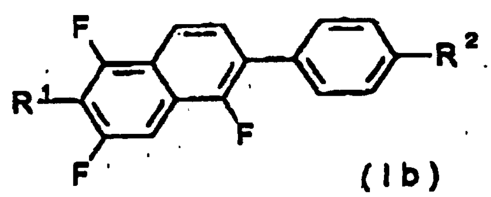
(Ib)

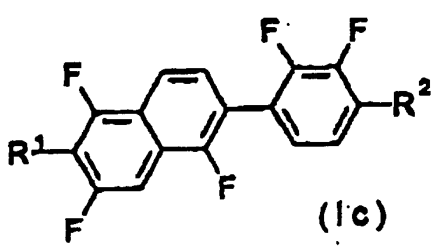
(Ic)

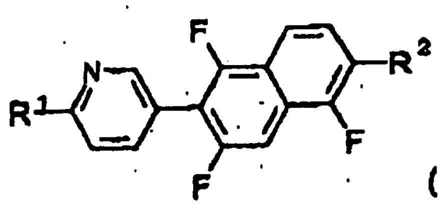
(Id)

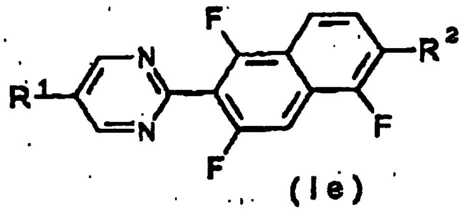
(Ie)

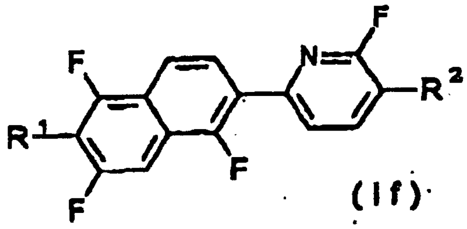
(If)

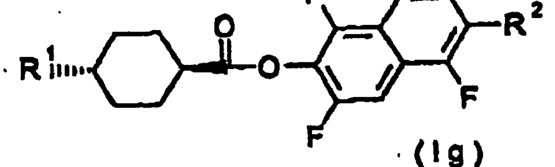
(Ig)

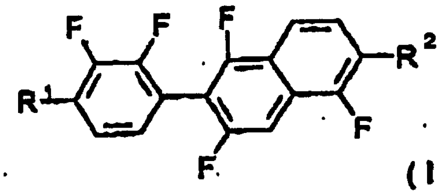
(Ih)

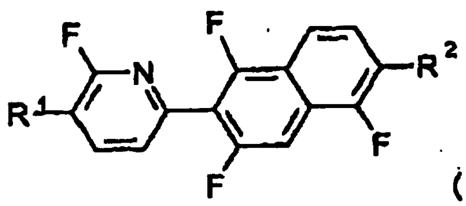
(Ii)

dans lequel $R^1$, $R^2$ ont les significations indiquées dans la revendication 1, avec la condition qu'au maximum un des groupes $R^1$, $R^2$ puisse être un atome d'hydrogène.

**3.** Utilisation de dérivés 1,3,5-trifluoronaphtalène selon une des revendications 1 ou 2 comme composants de mélanges de cristaux liquides.

**4.** Mélange de cristaux liquides, contenant un ou plusieurs dérivés 1,3,5-trifluoronaphtalène selon une des revendications 1 à 3.

**5.** Mélange de cristaux liquides selon la revendication 4, **caractérisé en ce qu'**il est ferroélectrique.

**6.** Mélange de cristaux liquides selon la revendication 4 ou 5, **caractérisé en ce qu'**il contient de 0,01 à 80 % en poids d'un ou de plusieurs dérivés 1,3,5-trifluoronaphtalène de formule (I).

**7.** Dispositif de commutation et/ou d'affichage ferroélectrique, contenant un mélange de cristaux liquides ferroélectriques selon la revendication 5 ou 6.

**8.** Dispositif de commutation et/ou d'affichage ferroélectrique selon la revendication 7, **caractérisé en ce qu'**il est actionné en mode $\tau V_{min}$.